# EUROPEAN PATENT APPLICATION

(11) **EP 3 153 522 A1**
(43) Date of publication of application: **12.04.2017**
(21) Application number: 16188974.6
(22) Date of filing: 15.09.2016
(51) Int. Cl.: C07K 14/505

(54) **PROCESS FOR THE PURIFICATION OF ERYTHROPOIETIN AND DARBEPOETIN ALFA**

(30) Priority: 09.10.2015 IN 5410CH2015; 27.04.2016 EP 16167237
(71) Applicant: Hetero Drugs Limited, 509301 Mahaboobnagar Telengana (IN)
(72) Inventor: Bandi, Vamsi Krishna, 500018 Hydrabad (IN); Reddy, Bala Reddy Bheema, 500018 Hydrabad (IN); Mugthihalli, Shivu Mokshanath, 500018 Hydrabad (IN); Iyer, Pradeep Kumar, 500018 Hydrabad (IN); Pasupuleti, Prasad, 500018 Hydrabad (IN)
(74) Representative: Kasche, André

(57) **Abstract**

The present invention is directed to a method for the purification of a protein, preferably a recombinant protein selected from the group consisting of erythropoietin, darbepoetin alfa, a functional fragment or a functional derivative of erythropoietin or darbepoetin alfa. Also, the present invention reads on a protein produced by said method and a corresponding pharmaceutical composition comprising said protein.

## Description

The invention provides sequential steps and conditions for the purification of proteins, preferably recombinant proteins selected from the group consisting of erythropoietin, darbepoetin alfa, a functional fragment or a functional derivative of erythropoietin or darbepoetin alfa, especially recombinant darbepoetin alfa.

### Background

Purification of recombinant proteins is the key to having an effective drug product. Erythropoietin and its analogues have sugar moieties that make up the glycan chains. These essentially contribute to the heterogeneity in the isoforms of these proteins, thus challenging the purification process. The sialic acid content in both erythropoietin and its analogue darbepoetin alpha determines the overall charge and isoelectric point (see Rush et al, Analytical Chemistry 1995, 67:1442). It is also widely reported that low pl isoforms exhibit higher bioactivity than those with high pl isoforms (see Zanette et al., Journal of Biotechnology, 2003, 101:275). In darbepoetin alpha, there is a heterogeneous mixture of isoforms with a pl ranging from 3 to 8 although the drug Aranesp (darbepoetin alpha) comprises low isoforms having a pl range of 3 to 3.9. Thus there is a need for better purification methods to address the heterogeneous mixtures of isoforms present in these protein.

An earlier method of erythropoietin purification described in WO198607594 involved reverse phase liquid chromatographic separation using immobilized C4 or C6 resin followed by elution of bound protein from the resin with aqueous ethanol solution at a pH of about 5 to 8. WO2003045996 discloses the purification of recombinant human erythropoietin by reverse phase chromatography followed by anion exchange chromatography and size exclusion chromatography.

WO2011063195 discloses a process to purify low pl isoforms of darbepoetin alpha from higher pl isoforms comprising at least one cation exchange chromatographic (CEX) step in bind-elute mode and one CEX step in flow-through mode. The above process allows for other intermediary anion exchange or mixed mode chromatographic steps before or after the two CEX steps.

All of the above processes, however, involve complex steps which may not achieve the desired percentage purity of the drug product. The present invention provides a method comprising sequential chromatographic and filtrations steps for the purification of proteins, preferably recombinant proteins selected from the group consisting of erythropoietin, darbepoetin alfa, a functional fragment or a functional derivative of erythropoietin or darbepoetin alfa erythropoietin, its analogues, especially darbepoetin alfa resulting in ≥ 98% pure drug substance.

### Figures

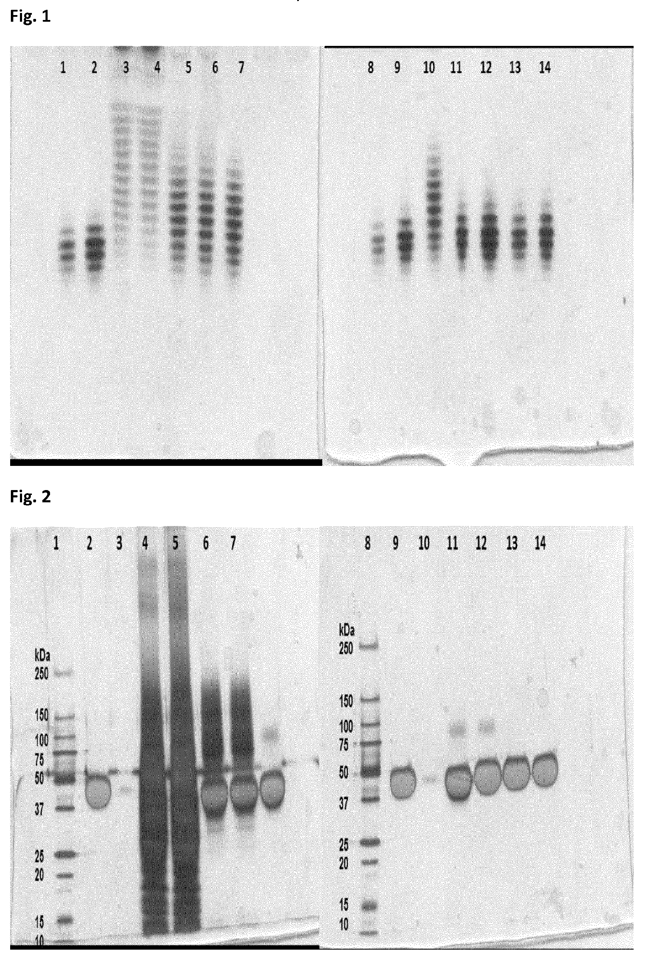

**Fig. 1** shows the results of an isoelectric focusing (IEF) analysis for isoform distribution. Lanes 1 and 8: 1 µg Standard (system suitability); Lanes 2 and 9: 5 µg Standard; Lane 3: anion exchange chromatography input - Bag 1; Lane 4: anion exchange chromatography input - Bag 2; Lane 5: anion exchange chromatography (I) eluate cycle 1; Lane 6: anion exchange chromatography I eluate cycle 2; Lane 7: anion exchange chromatography II eluate; Lane 10: anion exchange chromatography II eluate; Lane 11: cation exchange chromatography - Flow through; Lane 12: anion exchange chromatography III eluate; Lane 13: gel filtration chromatography - pooled; Lane 14: drug Substance.
**Fig. 2** shows an SDS-PAGE analysis for molecular weight related substances. Lanes 1 and 9: marker; Lanes 2 and 10: standard; Lanes 3 and 11: 1% standard; Lane 4: anion exchange chromatography input - Bag 1; Lane 5: anion exchange chromatography input - Bag 2; Lane 6: anion exchange chromatography I eluate cycle 1; Lane 7: anion exchange chromatography I eluate cycle 2; Lane 8: anion exchange chromatography II eluate; Lane 12: anion exchange chromatography II eluate; Lane 13: anion exchange chromatography III eluate; Lane 14: gel filtration chromatography - pooled; Lane 15: Drug Substance.

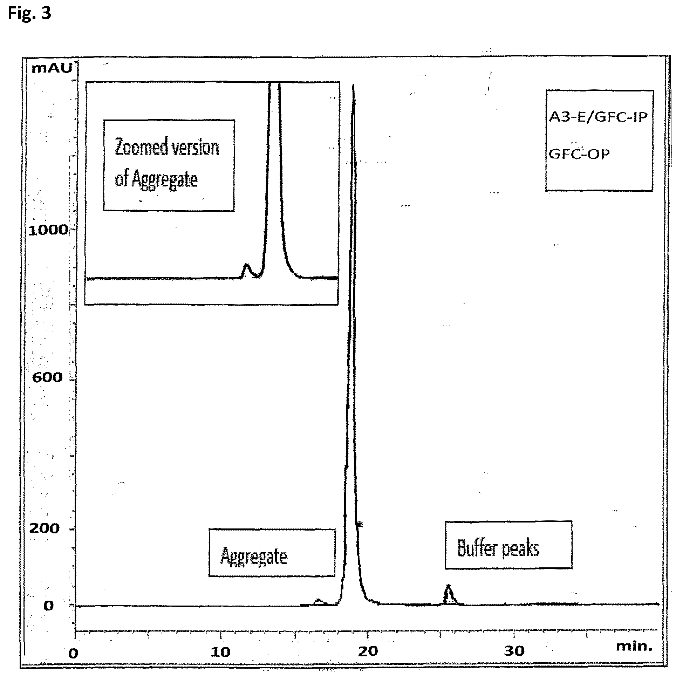

**Fig. 3** shows the analysis of product purity by size exclusion chromatography. A3-E/GFC-IP: Anion exchange chromatography III eluate/gel filtration chromatography - input; GFC-OP: Gel filtration chromatography - output.

The present invention provides a method for the purification of a protein, preferably a recombinant protein, selected from the group consisting of erythropoietin, darbepoetin alfa, a functional fragment or a functional derivative of erythropoietin or darbepoetin alfa, the method comprising the sequential chromatographic steps of:
(a) anion exchange chromatography (I); (b) mixed mode chromatography; (c) anion exchange chromatography (II); (d) cation exchange chromatography; (e) affinity chromatography; (f) anion exchange chromatography (III); (g) gel filtration chromatography.

The term "functional derivative" of erythropoietin or darbepoetin alfa is meant to include any polypeptide or fragment thereof that has been chemically or genetically modified in its amino acid sequence, e.g. by addition, substitution and/or deletion of amino acid residue(s) and/or has been chemically modified in at least one of its atoms and/or functional chemical groups, e.g. by additions, deletions, rearrangement, oxidation, reduction, etc. as long as the derivative still has at least some cytokine activity on erythropoiesis or red blood cell production to a measurable extent, e.g. a cytokine activity of at least about 1 to 10 % of at least one cytokine activity of human erythropoietin, e.g. on erythropoiesis and/or red blood cell production.

In this context a "functional fragment" of erythropoietin or darbepoetin alfa is one that forms part of erythropoietin or darbepoetin alfa or a derivative of these and still has at least some cytokine activity on erythropoiesis or red blood cell production to a measurable extent, e.g. a cytokine activity of at least about 1 to 10, at least 5 to 20, 10 to 50 % of at least one cytokine activity of human erythropoietin, e.g. on erythropoiesis and/or red blood cell production.

Preferably, a functional fragment or functional derivative for purification by the inventive method has a pl of 3 to 8 and/or about the same sialic acid content as human erythropoietin or darbepoetin alfa.

More preferably, a functional fragment or functional derivative for being purified by the inventive method has at least 60 or 70, preferably at least 80 or 90 %, more preferably at least 95 % sequence identity to the amino acid sequence of human erythropoietin or darbepoetin alfa.

Most preferably, a functional fragment or derivative of erythropoietin or darbepoetin alfa has at least one, preferably all of the following characteristics:
(i) a pl of 3 to 8,
(ii) about the same sialic acid content as human erythropoietin or darbepoetin alfa,
(iii) at least 80 or 90 %, more preferably at least 95 % sequence identity to the amino acid sequence of human erythropoietin or darbepoetin alfa, and/or
(iv) a cytokine activity of at least about 1 to 10, 5 to 20, 10 to 50 % of at least one cytokine activity of human erythropoietin, preferably on erythropoiesis and/or red blood cell production.

The identity of related amino acid molecules can be determined with the assistance of known methods. In general, special computer programs are employed that use algorithms adapted to accommodate the specific needs of this task. Preferred methods for determining identity begin with the generation of the largest degree of identity among the sequences to be compared. Preferred computer programs for determining the identity among two amino acid sequences comprise, but are not limited to, TBLASTN, BLASTP, BLASTX, TBLASTX (Altschul et al., (1990) J. Mol. Biol., 215, 403-410), ClustalW (Larkin MA et al., Bioinformatics, 23, 2947-2948, 2007) or PHYRE2 (Kelley LA et al., (2015) Nature Protocols 10, 845-858). The BLAST programs can be obtained from the National Center for Biotechnology Information (NCBI) and from other sources (BLAST handbook, Altschul et al., NCB NLM NIH Bethesda, MD 20894). The ClustalW program can be obtained from http://www.clustal.org and the PHYRE2 program from http://www.sbg.bio.ic.ac.uk/phyre2/html/page.cgi?id=index.

Preferably, the method of the invention is one, wherein
(i) anion exchange chromatography (I) is followed by buffer exchange;
(ii) mixed mode chromatography is followed by low pH treatment;
(iii) anion exchange chromatography (III) is followed by gel filtration chromatography; and
(iv) gel filtration chromatography is followed by nanofiltration.

In a preferred embodiment, the method of the present invention comprises chromatographic steps interspersed with buffer exchanges.

In another preferred embodiment the method of the invention provides for a low pH treatment at about 3.7 for about 1 hour after the step of mixed mode chromatography.

In a further preferred embodiment a buffer exchange is carried out at least in three stages:
(i) after anion exchange chromatography (I);
(ii) after anion exchange chromatography (II);
(iii) after affinity chromatography.

The method of purification disclosed herein preferably results in a protein which is at least about 98 percent pure (by weight), preferably at least 98.0 to 99.8 percent pure (by weight).

In a further aspect, the present invention relates to a protein selected from the group consisting of erythropoietin, darbepoetin alfa, a functional fragment or a functional derivative of erythropoietin or darbepoetin alfa produced by the above-described methods of the invention. A further aspect concerns a pharmaceutical composition comprising a protein of the invention and optionally physiologically acceptable pharmaceutical excipients.

### Detailed Description

In the following the method of the present invention is discussed by means of specific and preferred embodiments, none of which are to be considered limiting the scope of the invention further than the claims as appended. The preferably recombinant protein, for example, darbe-hjkpoetin alfa can be produced in Chinese Hamster Ovary (CHO) cells using appropriate culture conditions, temperature and pH.

The method of purification of the present invention can comprises the following steps:
(a) passing clarified filtered harvest through anion exchange chromatography (I) yielding eluate;
(b) eluate of (a) is subjected to buffer exchange I;
(c) the process of buffer exchange I is followed by loading onto mixed mode chromatography (MMC);
(d) MMC of (c) is followed by low pH treatment at pH 3.7 and readjustment to pH 4.5;
(e) step (d) is followed sequentially by anion exchange chromatography (II) and buffer exchange II;
(f) eluate of step (e) is loaded onto cation exchange chromatography;
(g) eluate of step (f) is loaded onto affinity chromatography step;
(h) affinity eluate of step (g) is subjected to buffer exchange III followed by anion exchange chromatography III;
(i) aggregates in the eluate from step (h) is filtered by gel filtration chromatography;
(j) the process of (i) is followed by nanofiltration to remove viruses.

The diluted drug product obtained from the above process has proven about 98.0 to 99.8% pure. In a preferred embodiment, recombinant darbepoetin alfa was purified by the method of the present invention to a purity of 99.8%.

In detail, the purification method of the present invention involves sequential steps as described below:
**(A) Anion Exchange Chromatography I:** This step, which is the initial step in the purification method, can be carried out with the clarified, filtered harvest resulting from the upstream process. In one embodiment, anion exchange chromatography (I) consists of Tris and NaCl at a pH of 7.2 which serves as equilibration and wash I buffer. Wash Buffer II consists of sodium acetate at pH 6.0. Wash buffer III consists of increased strength of sodium acetate at a pH of 3.7. The protein is further washed with wash buffer IV which also serves as pre-elution buffer using Tris at pH 7.2. Elution buffer consists of Tris and 200mM NaCl at a pH of 7.2. The protein is further subjected to regeneration buffer consisting of Tris and 1M NaCl at pH 7.2.
   In a preferred embodiment, the anion exchange chromatography (AEX-I) of the purification method is for the removal of almost 80% impurities. This step also facilitates lower column volumes for the following chromatography steps in addition to reducing the buffer volumes for the remaining steps of purification. Thus, AEX-I serves as a step for cost reduction in the downstream process.
   Also, 65% of the basic impurities are removed in flow through during AEX-I; wash III removes up to 10% of impurities; regeneration removes approximately 5% of impurities. Eluate following AEX, washings and regeneration leads to about collection of 20% of the loaded protein.
   It is envisaged that AEX-I step removes basic impurities, HCP, DNA, endotoxins and viruses. Buffer exchange-I is carried out for loading the AEX-I eluate on to the next step of mixed mode chromatography. The equilibration buffer used in this step is sodium acetate and sodium phosphate buffer at a pH of 6.0.
**(B) Mixed Mode Chromatography (MMC):** The mixed mode chromatography step in the purification method is performed to remove basic isoform impurities, HCP and DNA. This step involves use of equilibration and wash I buffer consisting of sodium acetate and sodium phosphate at pH 6.0 followed by regeneration consisting of regeneration buffer of 500 mM sodium phosphate at pH 6.8.
   In yet another embodiment, as a key step towards virus inactivation, low pH treatment is carried out at pH 3.7 for 1 hr. This step inactivates the enveloped viruses (≥4 Log₁₀Reduction Value, LRV). Tris is used to readjust the pH to 4.5.
**(C) Anion Exchange Chromatography II:** For further concentration of the protein and removal of HCP, DNA and all types of viruses (≥4 LRV), AEX-II is carried out. Further buffer exchange-II serves as an intermediary step between AEX-II eluate and loading of the protein onto cation exchange chromatography (CEX).
**(D) Cation Exchange Chromatography (CEX):** In a preferred embodiment, the CEX is performed to control the isoform profile, thus rendering the standard product quality. This step also removes HCP from the protein sample. The invention particularly provides the loading condition, such as pH, allowing higher resolution of isoforms as compared to routine cation exchange resins.
**(E) Affinity Chromatography:** In yet another embodiment the affinity chromatography step is carried out in the presence of equilibration buffer and elution buffer, each with different concentrations of NaCl to remove degraded protein that is generated in the CEX. Affinity eluate is conditioned with buffer exchange-III in order to load on to anion exchange chromatography (III) step.
**(F) Anion Exchange Chromatography III (AEX-III):** The invention provides yet another step of AEX which is primarily for concentration of the protein to be used in subsequent steps. Again, this is carried out in equilibration buffer (pH 6) and elution buffer (7.2).
**(G) Gel Filtration Chromatography:** In a preferred embodiment, gel filtration is used to remove aggregates. This step also provides for the protein product to be formulated in formulation buffer.
**(H) Nano Filtration:** This step is provided to remove all types of viruses (≥4 LRV) to satisfy regulatory requirement. Filtration after dilution results in drug substance which is put through bulk batch release tests and stability.

The resulting protein purified using the above sequential steps results in ≥98% purity. In a preferred embodiment, the protein purity achieved by this process in >99%. In a still preferred embodiment, the protein purity achieved by the said process is 99.8%.

It is provided that the purification method of the invention can be used for obtaining high purity drug product of proteins, especially recombinant proteins with higher sialic acid content. The invention provides especially for the purification of recombinant erythropoietin and darbepoetin alfa isoforms as well as functional fragments and derivatives thereof with similar or substantially the same sialic acid content and/or similar pl compared to the original erythropoietin or darbepoetin proteins. In a most preferred embodiment, the invention provides a method for the purification of recombinant darbepoetin alfa.

The examples given below further aid in the understanding of the invention. However, no aspect of the examples should be construed as limiting the scope of the invention.

### Method

### Upstream Process of obtaining Darbepoetin alfa protein:

One vial of Chinese Hamster Ovary (CHO) cells having Darbepoetin gene construct, producing Darbepoetin was thawed from liquid nitrogen transferred to shake flask culture with 40mL of culture medium and incubated at 37°C, 5 % CO₂ with 120 rpm agitation. After 3-4 days of incubation when the cell count reached to about 2-4 million cells/mL the cells were transferred and cultured in bigger flasks in shaker incubator at 37°C, 5 % CO₂ and 120 rpm. Subsequently, when the cell count reached 3-4 million cells/mL, these cells were transferred to seed bioreactor with a culture volume of 2L in a 6.5L glass bioreactor with an initial seeding density of 0.25 million cells/mL. The culture was agitated for 3-4 days till the cell count reached to about 2-4 million cells/mL and the process was shifted to production bioreactor with a seed density of 0.25 million cells/mL in 16L culture medium. Cells were grown in production bioreactor for 12 days with feeding on every alternate day starting from 3^{rd} day under set parameters of temperature at 37°C, dissolved oxygen at 50% and pH at 7.0±0.1 at varying agitation speeds depending upon oxygen demand. Culture medium with darbepoetin protein was harvested on Day 12 and clarified using 0.2µm hollow fiber to remove the cell debris.

### Example I

Darbepoetin harvested from the upstream process was subjected to the following process of purification

### A) Anion Exchange Chromatography I (AEX-I)

**Table 1. Buffer conditions for AEX-I**

| **Buffer** | **Concentration** | **pH** |
|---|---|---|
| Equilibration/Wash I buffer | 25mM Tris, 60mM NaCl | 7.2 |
| Wash II buffer | 21mM sodium acetate | 6.0 |
| Wash III buffer | 217mM sodium acetate | 3.7 |
| Pre-elution/Wash IV buffer | 25mM Tris | 7.2 |
| Elution Buffer | 25mM Tris, 200mM NaCl | 7.2 |
| Regeneration Buffer | 25mM Tris, 1M NaCl | 7.2 |

Buffer exchange I was performed for AEX-I eluate to load on to the next step of mixed mode chromatography. The equilibration buffer used in this step is 21mM sodium acetate and 5mM sodium phosphate buffer at a pH of 6.0.

**B) Mixed Mode Chromatography** was carried out with the following conditions given in Table 2.

**Table 2. Buffer conditions for Mixed Mode Chromatography (MMC)**

| **Buffer** | **Concentration** | **pH** |
|---|---|---|
| Equilibration/Wash I buffer | 21mM sodium acetate, 5mM sodium phosphate | 6.0 |
| Regeneration Buffer | 500mM sodium phosphate | 6.8 |

| | | |
|---|---|---|
| MMC accounted for removal of approximately 20% impurities. | | |

Low pH treatment at pH 3.7 for 1 hr was then carried out to inactivate the enveloped viruses. Tris at 1M was used to readjust pH to 4.5.

**(C) Anion Exchange Chromatography II: Conditions for AEX-II is provided in Table 3.**

**Table 3. Buffer Conditions for AEX-II**

| **Buffer** | **Concentration** | **pH** |
|---|---|---|
| Equilibration Buffer | 51mM sodium acetate | 4.5 |
| Elution Buffer | 25mM Tris, 200mM NaCl | 7.2 |
| Regeneration Buffer | 25mM Tris, 1M NaCl | 7.2 |

Buffer Exchange II was carried out with 51mM sodium acetate at pH 4.5.

**(D) Cation Exchange Chromatography (CEX):** To obtain protein purity, CEX was performed.

**Table 4: Conditions for CEX**

| **Buffer** | **Concentration** | **pH** |
|---|---|---|
| Equilibration Buffer | 51mM sodium acetate | 4.5 |
| Regeneration Buffer | 25mM Tris, 1M NaCl | 7.2 |

It was found that the conditions provided resulted in higher resolution of isoforms thus resulting in highly pure protein.

**(E) Affinity Chromatography:** This step was carried out with the following conditions (Table 5) in order to remove the degraded protein generated in CEX.

**Table 5.**

| **Buffer** | **Concentration** | **pH** |
|---|---|---|
| Equilibration Buffer | 21mM sodium acetate, 10mM NaCl | 6.0 |
| Elution Buffer | 21mM sodium acetate, 1M NaCl | 6.0 |

| | | |
|---|---|---|
| Buffer exchange III with 21mM sodium acetate at pH 6.0 was carried out. | | |

**(F) Anion Exchange Chromatography III (AEX-III):** Affinity chromatography was followed up with AEX-III in order to concentrate the protein prior to gel filtration. Conditions for AEX-III is provided below

**Table 6.**

| **Buffer** | **Concentration** | **pH** |
|---|---|---|
| Equilibration Buffer | 21mM sodium acetate | 6.0 |
| Elution Buffer | 25mM Tris, 200mM NaCl | 7.2 |

**(G) Gel Filtration Chromatography:** This step was carried out in formulation buffer at pH 6.2 resulting in removal of aggregates.

**(H) Nano Filtration:** As a final step before dilution and preparation of drug substance, nano filtration was carried out using 0.2µm filtration followed by 20nm filtration.

### Example 2

### Protein Purity Analysis

Darbepoetin alpha purified as per the steps of example 1 was analysed using isoelectric focusing for isoform distribution. The results clearly indicates removal of unrelated glycoforms across the various process steps (Figure 1). The final polishing is seen at the CEX where the isoform pattern is similar to that of innovator.

### Example 3

### Analysis for Molecular Weight related substances

SDS-PAGE was carried out to analyze the molecular weight of the eluates from different process steps (Figure 2). The results show reduction in low molecular weight impurities and high molecular weight impurities across the various process steps. Furthermore, removal of aggregates after the gel filtration chromatography step was seen with a purity of >99% and aggregation values less than 1%.

### Example 4

### Analysis of product purity by Size Exclusion Chromatography (SEC)

Fig. 3 shows anion exchange chromatography III eluate and Gel Filtration chromatography (A3-E/GFC-IP) input and Table 7 below provides the gel filtration chromatography output (GFC-OP).

**Table 7**

| **Sample** | **% HMW Impurities** | **% Purity** |
|---|---|---|
| A3-E/GFC-IP | 1.4 | 98.6 |
| GFC-OP | 0.2 | 99.8 |

The above results indicate quantitative reduction of aggregates in the GFC step as demonstrated by SEC.

## Claims

1. A method for the purification of a protein, preferably a recombinant protein selected from the group consisting of erythropoietin, darbepoetin alfa, a functional fragment or a functional derivative of erythropoietin or darbepoetin alfa, the method comprising the sequential chromatographic steps of:
(a) anion exchange chromatography (I);
(b) mixed mode chromatography;
(c) anion exchange chromatography (II);
(d) cation exchange chromatography;
(e) affinity chromatography;
(f) anion exchange chromatography (III);
(g) gel filtration chromatography.

2. The method of claim 1, wherein
(i) anion exchange chromatography (I) is followed by buffer exchange;
(ii) mixed mode chromatography is followed by low pH treatment;
(iii) anion exchange chromatography (III) is followed by gel filtration chromatography; and
(iv) gel filtration chromatography is followed by nanofiltration.

3. The method of claim 2, wherein the low pH treatment is at pH 3.7 for 1 hour.

4. The method of claim 1 wherein buffer exchange occurs after at least three stages:
(i) after anion exchange chromatography (I);
(ii) after anion exchange chromatography (II);
(iii) after affinity chromatography.

5. The method of claim 1, wherein the method results in a weight percentage purity of at least about 98, preferably of at least 98.0 to 99.8.

6. A protein selected from the group consisting of erythropoietin, darbepoetin alfa, a functional fragment or a functional derivative of erythropoietin or darbepoetin alfa produced by the method according to any of claims 1 to 5.
